# EUROPEAN PATENT APPLICATION

(11) **EP 4 458 383 A1**
(43) Date of publication of application: **06.11.2024**
(21) Application number: 22916387.8
(22) Date of filing: 07.11.2022
(51) Int. Cl.: A61M 1/00, A61B 17/34

(54) **CARTILAGE SEPARATION FILTER ASSEMBLY**

(30) Priority: 27.12.2021 KR 20210187937
(71) Applicant: Miracell Co., Ltd., Seoul 06222 (KR)
(72) Inventor: JI, Jung Ho, Seongnam-si Gyeonggi-do 13229 (KR); SHIN, Hyun Soon, Seoul 06295 (KR)
(74) Representative: Habermann, Hruschka & Schnabel
(86) International application number: PCT/KR2022/017300
(87) International publication number: WO 2023/128233

(57) **Abstract**

The present invention relates to a filter assembly for cartilage separation, and more particularly to a filter assembly for cartilage separation for separating cartilage from blood or the like discharged from an arthroscopic cannula during joint surgery with an arthroscope for reuse, A filter assembly for separating cartilage tissue mounted on a fluid drainage tube connected to a drainage valve attached to a cannula for arthroscopy, characterized in that the filter assembly for separating cartilage tissue comprises: an inlet three-way valve connected to the fluid drainage tube; a main tubing portion connected to the three-way valve to filter cartilage tissue; a bypass tubing portion connected to the three-way valve; and an outlet three-way valve connected to the main tubing portion and the bypass tubing portion.

## Description

### [Technical Field]

The present invention relates to a cartilage separation filter assembly, and more particularly, to a cartilage separation filter assembly to separate and reuse cartilage from blood that is connected to and discharged from an endoscopic cannula during joint surgery with an arthroscope.

### [Background Art]

In general, an endoscope is a device that is inserted into the inside of the human body to view the inside of the body to photograph the inside of the body or to perform a surgical procedure or surgery on the body. In the case of an arthroscope, a fluid supply tube and a fluid discharge tube in which fluid and blood are mixed are connected to the cannula during arthroscopy or joint surgery using an endoscope.

In conventional arthroscopic examinations and surgeries, cartilage that falls off from the joint is discarded through a fluid discharge tube, but since human-derived cartilage tissue can be easily implanted in the joint, reuse is required.

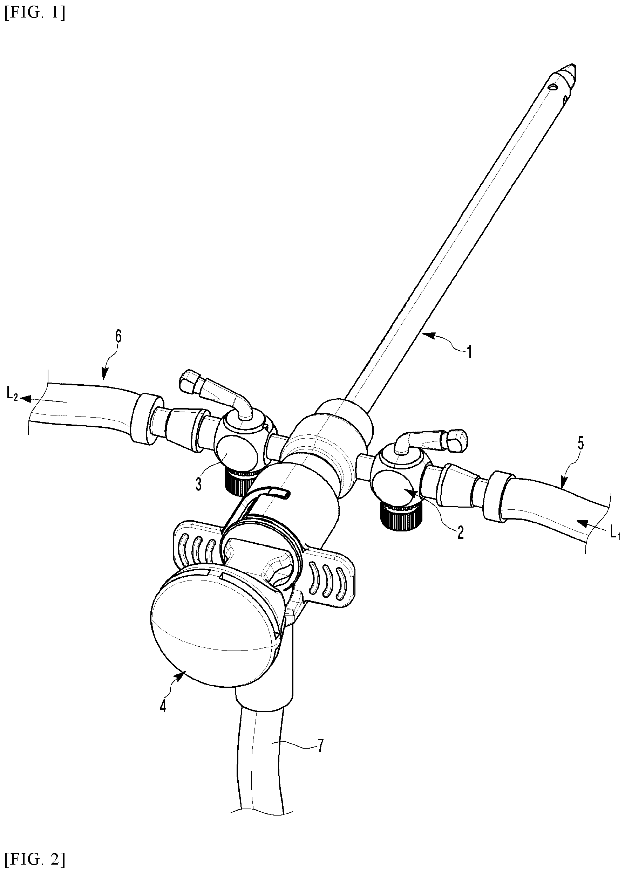

FIG. 1 is a drawing of an arthroscopic device, comprising a cannula 1 having a fluid supply valve 2 and an outlet valve 3 through which blood and fluid containing cartilage tissue are discharged, and a scope (4) inserted into said cannula 1, as shown in the drawing. Before attaching the scope (4), the cannula (1) is first inserted into the joint part using a trocar inserted into the cannula (1), and when the cannula (1) is inserted into the joint part, the trocar is removed and the scope (4) connected to the power and signal transmission cable (7) is inserted to inspect the joint part while photographing it in real time, and if necessary, surgery can be performed. Through the fluid supply tube (5) connected to the supply valve (3), fluid (L₁) is supplied to the joint part to be photographed, the joint part is washed with the fluid (L₁) supplied into the joint part, and the washed part is photographed or a surgical procedure or surgery is performed. The fluid (L₂) mixed with the washed fluid and blood containing cartilage tissue is discharged to the outside through the fluid discharge tube (6) through the discharge valve. The externally discharged fluid (L₂) mixed with cartilage tissue is stored in a storage container and then disposed of. As a result, reusable cartilage tissue may be discarded.

Therefore, there is a need for a tool or device for reusing the cartilage discharged along with the fluid during endoscopy or surgery.

### [Disclosure]

### [Technical Problem]

Accordingly, an object of the present invention is to solve the aforementioned problems of the prior art, and to provide a cartilage separation filter assembly that allows cartilage tissue that is drained along with fluid during arthroscopy or surgery to be separated from the fluid and reused.

### [Technical Solution]

To accomplish the above object, the present invention provides a cartilage separation filter assembly to separate cartilage tissue mounted on a fluid outlet tube connected to an outlet valve attached to a cannula for an arthroscope, the filter assembly comprising: an inlet three-way valve connected to the fluid outlet tube; a main tubing portion connected to the inlet three-way valve to filter cartilage tissue; a bypass tubing portion connected to the inlet three-way valve; and an outlet three-way valve connected to the main tubing portion and the bypass tubing portion.

In the present invention, the main tubing portion comprises: a main inlet tube connected with an inlet three-way valve; a filter portion connected with the main inlet tube; and a main outlet tube, the inlet side of which is connected with the filter portion and the outlet side of which is connected with the outlet three-way valve.

In the present invention, the filter portion comprises: a filter tube has a certain diameter such that the main inlet tube is inserted at a certain length on the inlet side and is attached to each other by thermal fusion, and the main outlet tube is inserted at a certain length on the outlet side and is attached to each other by thermal fusion; and, a filter which the front end is attached together by thermal fusion to form an inlet between the main inlet tube and the filter tube, and the rear end consists of a mesh net in the form of a pocket extending to a certain length for filtering cartilage tissue from a fluid containing blood and cartilage tissue flowing from the main inlet tube.

In the present invention, the inside of the filter further includes a first anti-deformation tube of filter tube for preventing the inlet side of the filter tube from being squeezed by the negative pressure, when the negative pressure is applied by a pump installed at the rear of the outlet three-way valve.

In the present invention, the inside of the filter tube further includes a second anti-deformation tube of filter tube installed at the rear of the filter to prevent the outlet side of the filter tube from being squeezed by the negative pressure.

Further, to accomplish the above object, the present invention provides a cartilage separation filter assembly to separate cartilage tissue mounted on a fluid outlet tube connected to an outlet valve attached to a cannula for an arthroscope, the filter assembly comprising: a Y-shaped inlet connection tube connected to the fluid outlet tube; a main tubing portion connected to the Y-shaped inlet connection tube and provided with a main valve to regulate the flow of fluid through the Y-shaped inlet connection tube to filter the cartilage tissue; a bypass tube portion connected to the Y-shaped inlet connection tube and provided with a bypass valve to regulate the flow of fluid; and a Y-shaped outlet connection tube to which the main tubing portion and the bypass tubing portion are connected.

In the present invention, the main tubing portion comprises: a main inlet tube connected to the Y-shaped inlet connection tube and provided with a main valve; a filter portion connected to the main inlet tube; and a main outlet tube, the inlet side of which is connected to the filter portion and the outlet side of which is connected to the Y-shaped outlet connection tube.

In the present invention, the filter portion comprises: a filter tube has a certain diameter such that the main inlet tube is inserted at a certain length on the inlet side and is attached to each other by thermal fusion, and the main outlet tube is inserted at a certain length on the outlet side and is attached to each other by thermal fusion; and, a filter which the front end is attached together by thermal fusion to form an inlet between the main inlet tube and the filter tube, and the rear end consists of a mesh net in the form of a pocket extending to a certain length for filtering cartilage tissue from a fluid containing blood and cartilage tissue flowing from the main inlet tube.

In the present invention, the inside of the filter further includes a first anti-deformation tube of filter tube for preventing the inlet side of the filter tube from being squeezed by the negative pressure, when the negative pressure is applied by a pump installed at the rear of the Y-shaped outlet connection tube.

In the present invention, the inside of the filter tube further includes a second anti-deformation tube of filter tube installed at the rear of the filter to prevent the outlet side of the filter tube from being squeezed by the negative pressure.

### [Advantageous Effects]

The present invention has the advantage that the cartilage tissue discharged with the fluid during arthroscopy or surgery can be separated from the fluid and reused.

Furthermore, the present invention has the advantage that the filter tube is not squeezed even when negative pressure is applied due to pump operation, enabling smooth fluid flow.

Furthermore, the present invention has the advantage of being equipped with a bypass tube, so that the fluid can be discharged directly when it is not necessary to separate the cartilage tissue.

### [Description of Drawings]

FIG. 1 is a perspective view of a conventional endoscopic cannula.
FIG. 2 is a perspective view of a first embodiment of a cartilage separation filter assembly according to the present invention.
FIG. 3 is a perspective view of a second embodiment of a cartilage separation filter assembly according to the present invention.
FIG. 4 is a perspective view of the filter portion of a cartilage separation filter assembly according to the present invention.
FIG. 5 is a partially cut perspective view of the filter portion of a cartilage separation filter assembly according to the present invention.
FIG. 6 is a partially cut perspective view of a filter of a cartilage separation filter assembly according to the present invention.
FIG. 7 is a cutaway perspective view of the filter of FIG. 6.
FIG. 8 is a cartilage recovery diagram showing the steps of recovering cartilage tissue from a cartilage separation filter assembly according to the present invention.

### [Best Mode for Disclosure]

The best mode for disclosure of the present invention is composed of a cartilage separation filter assembly to separate cartilage tissue mounted on a fluid outlet tube connected to an outlet valve attached to a cannula for an arthroscope, the filter assembly comprising: an inlet three-way valve connected to the fluid outlet tube; a main tubing portion connected to the inlet three-way valve to filter cartilage tissue; a bypass tubing portion connected to the inlet three-way valve; and an outlet three-way valve connected to the main tubing portion and the bypass tubing portion.

### [Mode for Disclosure]

Hereinafter, preferred embodiments of the present invention are described in detail with reference to the accompanying drawings, which are intended to be sufficiently detailed to enable one having ordinary skill in the art to practice the invention, but are not intended to limit the technical ideas and scope of the present invention.

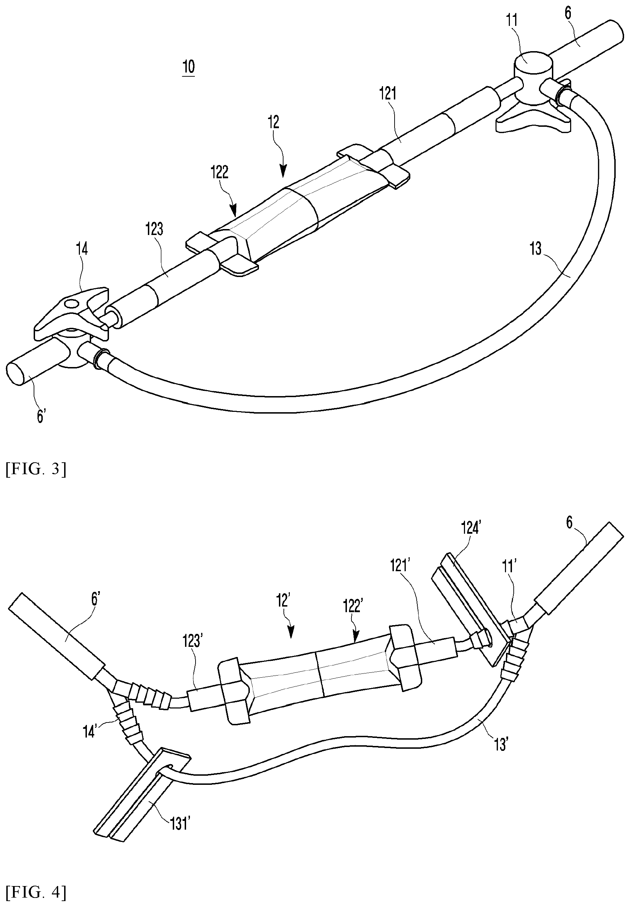

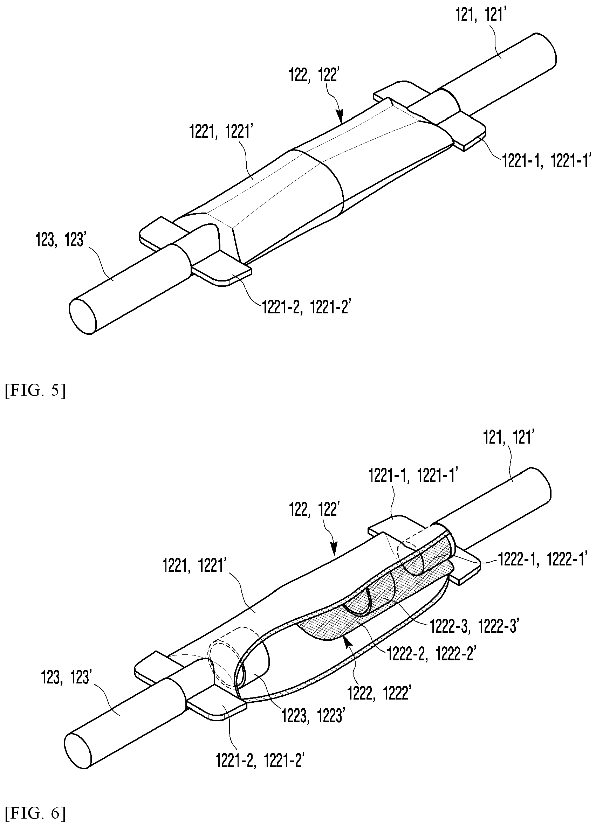

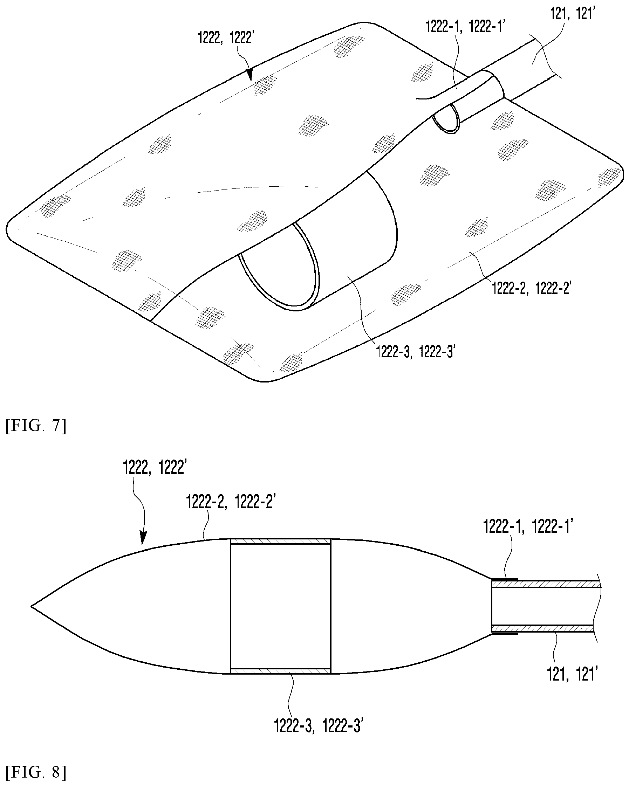

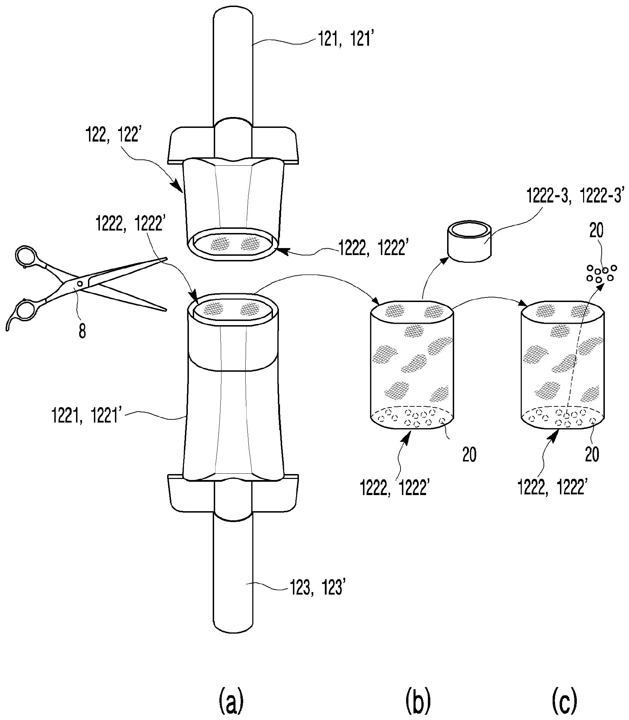

FIG. 1 is a perspective view of a conventional endoscopic cannula, FIG. 2 is a perspective view of a first embodiment of a cartilage separation filter assembly according to the present invention, FIG. 3 is a perspective view of a second embodiment of a cartilage separation filter assembly according to the present invention, FIG. 4 is a perspective view of the filter portion of a cartilage separation filter assembly according to the present invention, FIG. 5 is a partially cut perspective view of the filter portion of a cartilage separation filter assembly according to the present invention, FIG. 6 is a partially cut perspective view of a filter of a cartilage separation filter assembly according to the present invention, FIG. 7 is a cutaway perspective view of the filter of FIG. 6, and FIG. 8 is a cartilage recovery diagram showing the steps of recovering cartilage tissue from a cartilage separation filter assembly according to the present invention.

In FIG. 1, an arthroscopic cannula 1 is inserted into a joint portion, a scope 4 is attached to the cannula 1, and the joint portion is imaged through the scope 4. In order to film the joint with the scope 4, it is necessary to wash the joint with fluid. Accordingly, the fluid (L₁) supplied from the fluid supply tube (5) is injected into the joint through the cannula 1 by opening the fluid supply valve 2. After washing, the fluid is discharged again through the cannula (1) to the fluid outlet tube (6). The fluid that washes the joint part may include blood and cartilage tissue, and the fluid (L₂) mixed with these various substances is discharged through the fluid outlet tube (6) by opening the fluid outlet valve 3. The present invention presents a method for separating cartilage tissue from the fluid (L₂) mixed with cartilage tissue and the like and reusing it.

FIG. 2 is a perspective view of a first embodiment (10) of a cartilage separation filter assembly according to the present invention. Referring to FIG. 2 and FIGS. 4 to 7, the first embodiment (10) of a cartilage separation filter assembly according to the present invention will be described in detail.

As shown in FIG. 2, the cartilage separation filter assembly (10) comprises an inlet three-way valve (11) connected to a fluid outlet tube (6), a main tube portion (12) connected to the inlet three-way valve (11) to filter cartilage tissue, a bypass tube (13) connected to the inlet three-way valve (11), and an outlet three-way valve (14) connected to the main tube portion (12) and the bypass tube (13).

As shown in FIGS. 4 and 5, the main tube portion (12) comprises a main inlet tube (121) connected to the inlet three-way valve (11), a filter portion (122) connected to the main inlet tube (121), and a main outlet tube (123) connected to the filter portion (122) on the inlet side and to the outlet three-way valve (14) on the outlet side. The filter portion (122) is formed to be larger than the diameters of the main inlet tube (121) and the main outlet tube (123) so that the main inlet tube (121) and the main outlet tube (123) can be inserted and thermally fused together and attached to each other, as shown in the drawing. Specifically, the filter portion (122) comprises a filter tube (1221) having a certain diameter so that the main inlet tube (121) is inserted at a certain length on the inlet side and attached to each other by thermal fusion, and the main outlet tube (123) is inserted at a certain length on the outlet side and attached to each other by thermal fusion, and a filter(1222) between the main inlet tube (121) and the filter tube (1221), the front side of which is attached together by thermal fusion to form an inlet (1222-1) and the rear side of which consists of a pocket-shaped mesh net (1222-2) extending to a certain length to filter cartilage tissue from the fluid (L₂) containing blood and cartilage tissue flowing from the main inlet tube (121). The filter tube (1221) is formed with a diameter larger than the diameter of the main inlet tube (121) and the main outlet tube (123), and the main inlet tube (121) and the main outlet tube (123) are inserted into the front and rear of the filter tube (1221) for a certain length and then attached to each other by thermal fusion to form an inlet side adhesive portion (1221-1) by the main inlet tube (121) and an outlet side adhesive portion (1221-2) by the main outlet tube (123).

As shown in FIG. 5, the filter (1222) is inserted into the inner side of the filter tube (1221) and then thermally fused together between the main inlet tube (121) and the filter tube (1221). Accordingly, the filter (1222) forms an inlet (1222-1) by the main inlet tube (121) and a pocket-shaped mesh net (1222-2) to filter the cartilage tissue contained in the fluid (L₂). A pump (not shown) may be attached to the end of the fluid outlet tube (6), and when the pump is driven, a negative pressure may be applied to the filter tube (1221) by the pressure acting through the fluid outlet tube (6) because the diameter of the filter tube (1221) is larger than the diameter of the fluid outlet tube (6). If negative pressure is applied to the filter tube (1221), the filter tube (1221) may be squeezed, and if the filter tube (1221) is squeezed, the fluid cannot be discharged smoothly through the filter tube (1221). Accordingly, a first anti-deformation tube (1223) of filter tube of a certain length formed in the form of a circular tube is inserted inside the filter tube (1221) so that deformation does not occur even when a negative pressure is applied to the filter tube (1221). The first anti-deformation tube (1223) of filter tube is provided on the outlet side to prevent squeezing of the filter tube on the outlet side.

FIGS. 6 and 7 illustrate a filter (1222). The filter (1222) is made up of a pocket-shaped mesh network of a certain mesh, which is thermally fused together after the main inlet tube (121) is inserted into the filter (1222) to form an inlet (1222-1) by the main inlet tube (121). A pump (not shown) may be attached to the end of the fluid outlet tube (6), and when the pump is driven, a negative pressure may be applied to the filter tube (1221) by the pressure acting through the fluid outlet tube (6) because the diameter of the filter tube (1221) is larger than the diameter of the fluid outlet tube (6). If negative pressure is applied to the filter tube (1221), the filter tube (1221) may be squeezed, and if the filter tube (1221) is squeezed, the fluid cannot be discharged smoothly through the filter tube (1221). Accordingly, in order to prevent deformation from occurring when negative pressure is applied to the filter tube (1221), a second anti-deformation tube (1222-3) of filter tube is inserted inside the filter (1222), and the second anti-deformation tube (1222-3) of filter tube prevents deformation of the filter tube on the inlet side.

FIG. 3 is a perspective view of a second embodiment (10') of a cartilage separation filter assembly according to the present invention. Referring to FIG. 3 and FIGS. 4 to 7, the second embodiment (10') of the cartilage separation filter assembly according to the present invention will be described in detail.

As shown in FIG. 3, the cartilage separation filter assembly (10') includes a Y-shaped inlet connection tube (11') connected to the fluid outlet tube (6), a main tube portion (12') connected to the Y-shaped inlet connection tube (11') and equipped with a main valve (124') to regulate the flow of fluid (L₂) through the Y-shaped inlet connection tube (11') to filter cartilage tissue, a bypass tube portion (13') connected to the Y-shaped inlet connection tube (11') and provided with a bypass valve (13 1') to regulate the flow of the fluid (L₂), and a Y-shaped outlet connection tube (14') to which the main tube portion (12') and the bypass tube portion (13') are connected.

As shown in FIGS. 4 and 5, the main tube portion (12') comprises a main inlet tube (121') connected to the Y-shaped inlet connection (11'), a filter portion (122') connected to the main inlet tube (121'), and a main outlet tube (123') connected to the filter portion (122') on the inlet side and to the Y-shaped outlet connection tube (14') on the outlet side. The filter portion (122') is formed larger than the diameters of the main inlet tube (121') and the main outlet tube (123') so that the main inlet tube (121') and the main outlet tube (123') can be inserted and thermally fused together and attached to each other, as shown in the drawing. Specifically, the filter portion (122') comprises a filter tube (1221') having a certain diameter so that the main inlet tube (121') is inserted at a certain length on the inlet side and is attached to each other by thermal fusion and the main outlet tube (123') is inserted at a certain length on the outlet side and is attached to each other by thermal fusion, and a filter (1222) between the main inlet tube (121') and the filter tube (1221'), the front side of which is attached together by thermal fusion to form an inlet (1222-1') and the rear side of which consists of a pocket-shaped mesh net (1222-2') extending to a certain length to filter cartilage tissue from the fluid (L₂) containing blood and cartilage tissue flowing in from the main inlet tube (121'). The filter tube (1221') is formed larger than the diameter of the main inlet tube (121') and the main outlet tube (123'), and the main inlet tube (121') and the main outlet tube (123') are attached to each other by thermal fusion after a certain length is inserted into the front and rear of the filter tube (1221') to form an inlet side adhesive portion (1221-1') by the main inlet tube (121') and an outlet side adhesive portion (1221-2') by the main outlet tube (123').

As shown in FIG. 5, the filter (1222') is inserted into the inner side of the filter tube (1221') and then thermally fused together between the main inlet tube (121') and the filter tube (1221'). Accordingly, the filter (1222') forms an inlet (1222-1') by the main inlet tube (121') and a pocket-shaped mesh net (1222-2') to filter cartilage tissue contained in the flowing fluid (L₂). A pump (not shown) may be attached to the end of the fluid outlet tube (6), and when the pump is driven, a negative pressure may be applied to the filter tube (1221') by the pressure acting through the fluid outlet tube (6) because the diameter of the filter tube (1221') is larger than the diameter of the fluid outlet tube (6). If negative pressure is applied to the filter tube (1221'), the filter tube (1221') may be squeezed, and if the filter tube (1221') is squeezed, the fluid cannot be discharged smoothly through the filter tube (1221'). Accordingly, a first anti-deformation tube(1223') of filter tube of a certain length formed in the form of a circular tube is inserted inside the filter tube (1221') so that deformation does not occur even when a negative pressure is applied to the filter tube (1221'). The first anti-deformation tube (1223') of filter tube is provided on the outlet side to prevent squeezing of the filter tube on the outlet side.

FIGS. 6 and 7 illustrate a filter (1222'). The filter (1222') is made of a pocket-shaped mesh net of a certain mesh, which is thermally fused together after the main inlet tube (121') is inserted into the filter (1222') to form the inlet (1222-1') by the main inlet tube (121'). A pump (not shown) may be attached to the end of the fluid outlet tube (6), and when the pump is driven, a negative pressure may be applied to the filter tube (1221') by the pressure acting through the fluid outlet tube (6) because the diameter of the filter tube (1221') is larger than the diameter of the fluid outlet tube (6). If negative pressure is applied to the filter tube (1221'), the filter tube (1221') may be squeezed, and if the filter tube (1221') is squeezed, the fluid cannot be discharged smoothly through the filter tube (1221'). Accordingly, in order to prevent deformation from occurring when the filter tube (1221') is subjected to negative pressure, a second anti-deformation tube (1222-3') of filter tube is inserted inside the filter (1222'), and the second anti-deformation tube (1222-3') of filter tube prevents deformation of the filter tube on the inlet side.

FIG. 8 illustrates a process for recovering the cartilage tissue (20) when the cartilage tissue (20) is collected by the filters (1222, 1222') of the filter portions (122, 122'). FIG. 8(a) illustrates cutting the filter tubes (1221, 1221') of the filter portions (122, 122') with scissors (8). Since the cartilage tissue (20) in the filter portion (122, 122') needs to be collected externally, the filter (1222, 1222') is collected by cutting with the scissors (8). As shown in FIG. 8(b), the second anti-deformation tube (1222-3, 1222-3') of filter tube is first removed from the collected filter (1222, 1222'). After the second anti-deformation tubes (1222-3, 1222-3') are removed, the cartilage tissue (20) contained in the filters (1222, 1222') can be retrieved using a tool, and the retrieved cartilage tissue (20) can be reintroduced into the articulating portion for reuse.

Although several embodiments have been described above by way of example, it will be apparent to one of ordinary skill in the art that the invention may be embodied in many other forms without departing from the spirit and scope thereof. Accordingly, the embodiments described above should be considered exemplary rather than limiting, and all embodiments within the scope of the appended claims and equivalents thereof will be the to be within the scope of the invention.

### [Industrial applicability]

The present invention relates to a cartilage separation filter assembly for separating cartilage from blood or the like discharged by being connected to an arthroscopic cannula during joint surgery with an arthroscopic, and is highly industrial in use.

## Claims

1. A cartilage separation filter assembly to separate cartilage tissue mounted on a fluid outlet tube connected to an outlet valve attached to a cannula for an arthroscope, the cartilage separation filter assembly comprising:
an inlet three-way valve connected to the fluid outlet tube;
a main tubing portion connected to the inlet three-way valve to filter cartilage tissue;
a bypass tubing portion connected to the inlet three-way valve; and
an outlet three-way valve connected to the main tubing portion and the bypass tubing portion.

2. The cartilage separation filter assembly of claim 1,
wherein the main tubing portion comprises:
a main inlet tube connected with an inlet three-way valve; a filter portion connected with the main inlet tube; and
a main outlet tube, the inlet side of which is connected with the filter portion and the outlet side of which is connected with the outlet three-way valve.

3. The cartilage separation filter assembly of claim 2,
wherein the filter portion comprises:
a filter tube has a certain diameter such that the main inlet tube is inserted at a certain length on the inlet side and is attached to each other by thermal fusion, and the main outlet tube is inserted at a certain length on the outlet side and is attached to each other by thermal fusion; and,
a filter which the front end is attached together by thermal fusion to form an inlet between the main inlet tube and the filter tube, and the rear end consists of a mesh net in the form of a pocket extending to a certain length for filtering cartilage tissue from a fluid containing blood and cartilage tissue flowing from the main inlet tube.

4. The cartilage separation filter assembly of claim 3,
wherein the inside of the filter further includes a first anti-deformation tube of filter tube for preventing the inlet side of the filter tube from being squeezed by the negative pressure, when the negative pressure is applied by a pump installed at the rear of the outlet three-way valve.

5. The cartilage separation filter assembly of claim 4,
wherein the inside of the filter tube further includes a second anti-deformation tube of filter tube installed at the rear of the filter to prevent the outlet side of the filter tube from being squeezed by the negative pressure.

6. A cartilage separation filter assembly to separate cartilage tissue mounted on a fluid outlet tube connected to an outlet valve attached to a cannula for an arthroscope, the cartilage separation filter assembly comprising:
a Y-shaped inlet connection tube connected to the fluid outlet tube;
a main tubing portion connected to the Y-shaped inlet connection tube and provided with a main valve to regulate the flow of fluid through the Y-shaped inlet connection tube to filter the cartilage tissue;
a bypass tube portion connected to the Y-shaped inlet connection tube and provided with a bypass valve to regulate the flow of fluid; and
a Y-shaped outlet connection tube to which the main tubing portion and the bypass tubing portion are connected.

7. The cartilage separation filter assembly of claim 6,
wherein the main tubing portion comprises:
a main inlet tube connected to the Y-shaped inlet connection tube and provided with a main valve;
a filter portion connected to the main inlet tube; and
a main outlet tube, the inlet side of which is connected to the filter portion and the outlet side of which is connected to the Y-shaped outlet connection tube.

8. The cartilage separation filter assembly of claim 7,
wherein the filter portion comprises:
a filter tube has a certain diameter such that the main inlet tube is inserted at a certain length on the inlet side and is attached to each other by thermal fusion, and the main outlet tube is inserted at a certain length on the outlet side and is attached to each other by thermal fusion; and,
a filter which the front end is attached together by thermal fusion to form an inlet between the main inlet tube and the filter tube, and the rear end consists of a mesh net in the form of a pocket extending to a certain length for filtering cartilage tissue from a fluid containing blood and cartilage tissue flowing from the main inlet tube.

9. The cartilage separation filter assembly of claim 8,
wherein the inside of the filter further includes a first anti-deformation tube of filter tube for preventing the inlet side of the filter tube from being squeezed by the negative pressure, when the negative pressure is applied by a pump installed at the rear of the outlet three-way valve.

10. The cartilage separation filter assembly of claim 9,
wherein the inside of the filter tube further includes a second anti-deformation tube of filter tube installed at the rear of the filter to prevent the outlet side of the filter tube from being squeezed by the negative pressure.
